(19) Europäisches Patentamt
European Patent Office
Office européen des brevets

(11) Publication number: **0 483 803 A1**

# EUROPEAN PATENT APPLICATION

(21) Application number: **91118521.3**

(22) Date of filing: **30.10.91**

(51) Int. Cl.5: **A61M 25/06**

(30) Priority: **01.11.90 JP 297006/90**

(43) Date of publication of application:
**06.05.92 Bulletin 92/19**

(84) Designated Contracting States:
**BE DE FR GB IT SE**

(71) Applicant: **JUNKOSHA CO. LTD.**
**25-25, Miyasaka 2-chome**
**Setagaya-ku Tokyo 156(JP)**

(72) Inventor: **Ikeda, Shingo**
**Nakayama 393**
**Hannoh, Saitama(JP)**
Inventor: **Kojima, Hiroshi**
**Ohaza Kawadera 611**
**Hannoh, Saitama(JP)**

(74) Representative: **Kehl, Günther, Dipl.-Phys. et al**
**Patentanwälte HAGEMANN & KEHL**
**Ismaninger Strasse 108**
**W-8000 München 80(DE)**

(54) **Tubes for introducing medical devices.**

(57) A tube 2 for introducing percutaneously a tubular or a wire-shaped medical device such as a catheter 20 into a living body, said tube for introducing medical devices being such which first introduces the medical device by bearing the device into the hollow portion thereof and which is then removed by tearing off, and being characterized by that it is made of a silicone - polyimide copolymer resin. The present invention provides a tube 2 which can be easily parted along the longitudinal direction thereof.

Fig. 2

## BACKGROUND OF THE INVENTION

### 1. Field of the Invention

The present invention relates to a synthetic resin tube for introducing and placing tubular or linear medical devices such as catheters and wire guides into the bodies of organisms.

### 2. Description of the Prior Art

Catheters made of flexible synthetic resin materials are widely used nowadays to inject percutaneously nutritive agents and drugs into blood vessels of patients. Since the tips of such catheters cannot be directly inserted into vascular systems, the catheters are generally introduced and placed inside a living body following a procedure comprising:

preparing first a synthetic resin tube as an introducing means for the catheters, said tube having an inner diameter roughly corresponding to the outer diameter of the catheter to be inserted;

fixing an injection syringe to the tube in such a manner that the needle tip of the syringe may partly stick out from the front end of said tube;

injecting the needle and the tube in the arrangement above to a blood vessel, and pulling out the syringe while leaving the tube inside the blood vessel; and

inserting immediately thereafter the catheter to introduce the tip thereof to the blood vessel.

Once the synthetic resin tube is used for establishing the catheter in the blood vessel, it not only becomes unnecessary, but also becomes an obstruction to the subsequent medical treatments. Thus, the used synthetic resin tube after installation of a catheter is generally torn in the longitudinal direction thereof to remove it off from the blood vessel. To facilitate this removal by making it easier to tear the resin tube off along the longitudinal direction, there have been proposed, for example, a method which comprises providing a strip of a material different from that of the tube along the longitudinal direction of the tube, as disclosed in JP-A-62-176459, JP-A-46476, and the like (the term "JP-A-" used herein signifies an "unexamined published Japanese patent application"), and methods which comprise providing a brittle portion partly in the tube wall, such as rendering the tube material heterogeneous by irradiating a radiation along the longitudinal direction of the tube and intentionally incorporating a weak portion along the peripheral direction of the tube by modifying the die into a particular structure, as proposed in JP-A-59-91967 and JP-A-2-177966.

However, the former methods which comprise providing continuously a strip along the longitudinal direction of the tube, said strip made of a material different from that of the tube wall, not only requires the installation of an expensive extrusion machine having a complicated structure for the mixing and placing of a second synthetic resin, but also suffers poor productivity ascribed to the difficulty in speeding up the molding rate. The latter methods which comprise establishing a brittle portion in the tube wall, also, require a special equipment or a special die. Moreover, since all the tubes thus produced by the conventional methods have a uniform appearance, the position of a hub which is attached to the tube end for tearing the tube open needs to be aligned with the tearing position of the tube. This alignment process, however, accompanies difficulty because of the poor distinctness of the positions and hence impairs operability at the production.

## SUMMARY OF THE INVENTION

An object of the present invention is, in the light of the circumstances mentioned hereinbefore, to provide a tube for introducing medical devices, which can be produced simply by extrusion without using a specially designed apparatus, and which is capable of being torn off at any position in the peripheral direction thereof so that the hub may easily be attached thereto.

Accordingly, the present inventors studied extensively to overcome the earlier difficulties and found that the use of a particular synthetic resin can provide a solution to the problems. That is, the foregoing object and others can be achieved by the present invention which provides a tube for introducing a tubular or a wire-shaped medical device such as a catheter into a living body, said tube for introducing medical devices being such which first introduces the medical device by bearing the device into the hollow portion thereof and which is then removed by tearing it off, characterized by that it is made of a silicone - polyimide copolymer resin.

The silicone - polyimide copolymer resin which is used as the material of the tube for introducing medical devices is a synthetic resin capable of being molded by melt extrusion, however, the present inventors have found, as a distinguishing characteristics of this resin, that the molecular chains thereof easily become oriented under some particular molding conditions, for example, when it is molded by increasing the drawdown immediately after the discharge thereof from the extruder die. The reason why such a distinct characteristic is inherent particularly in the silicone - polyimide copolymer resin is yet to be clarified. Generally, in the case of a resin molding comprising one-direction oriented molecular chains, the mechanical properties such as tensile strength along the direction perpendicular to the direction of the molecular orientation tend to be impaired. Thus, in general, molding conditions are selected to avoid such molecular orientation. The present invention positively takes advantage of such preferential orientation of the molecular chains however, and, the present invention is characterized by this point.

More specifically, the present invention induces the chain molecules of the silicone - polyimide copolymer resin to take a highly oriented arrangement along the extruded direction. The resulting tube comprises less entangled copolymer resin chain molecules which are regularly arranged along the longitudinal direction of the tube. Thus, the mechanical strength of the tube along the tube peripheral is considerably lowered and hence the tube can be easily torn along the longitudinal direction. Since this property appears equally at any place in the tube peripheral, there is no need to strictly search the position of the tube end for attaching the hub. Accordingly, the operability is considerably improved.

BRIEF DESCRIPTION OF THE DRAWINGS

FIG. 1 is a frontal view of an embodiment of the tube for introducing medical devices according to the present invention; and
FIG. 2 is a schematic view which illustrates the mode of usage of the tube for introducing medical devices as shown in FIG. 1.

DETAILED DESCRIPTION OF THE PREFERRED EMBODIMENT

An embodiment of the present invention is explained in further detail below referring to the drawings, but it should be understood that the present invention is not to be construed as being limited thereto.

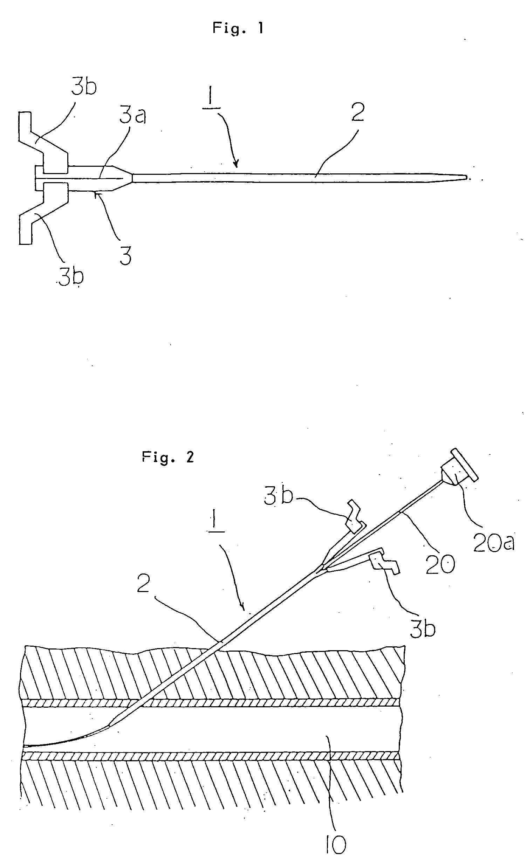

Referring to FIG. 1, a frontal view of a tube for introducing medical devices according to the present invention is shown. The tube 1 comprises a tube body 2 having a tapered front end, made of a silicone - polyimide copolymer resin (e.g., SILTEM (Trade Name), manufactured by General Electric Co.). In this Example, to the base end side of the tube body 2 is further fixed a cylindrical hub 3 having provided on the wall periphery thereof two slits 3a facing each other, each slit being provided in parallel to the center axis and being extended from one end of the cylinder to the other end.

The tube body 2 is produced by extrusion molding in such a manner that the molecules thereof be highly oriented along the longitudinal direction of the tube, for example, by increasing the drawdown immediately after discharge from the die, or by making the length of the land portion of the die longer than that of a commonly used die. Thus, the tube body 2 can be torn along the longitudinal direction of the tube from any part of the tube periphery.

Referring then to FIG. 2, the mode of usage of the tube for introducing medical devices described with reference to FIG. 1 is explained below. A syringe (not shown in the FIGURE) is inserted into the hub 3 being fixed to the base end of the tube body 2, and the whole tube for introducing medical devices is injected to a blood vessel 10 with the needle tip sticking out from the front end of the tube body 2. The syringe together with the needle are drawn out of the tube body 2, and a catheter 20 is immediately inserted through the hub 3 to the tube body 2, until the front tip thereof intrudes into the blood vessel 10.

Then, as is shown in the figures the hub 3 is opened along the slits 3a by taking hold on the protruded portions 3b, so that the tube body 2 may be pulled out from the blood vessel 10. Since the material constituting the tube wall is rendered molecular orientation, the tube body 2 can be easily parted along the longitudinal direction thereof with the opening of the hub by the protruded portions 3b. Thus, the tube for introducing medical devices 1 is greatly contributory to introducing medical devices into live bodies, since the tube for introducing medical devices 1 can be easily detached from the blood vessel 10 and the catheter 20 without being obstructed by the enlarged portion 20a provided at the base end side of the catheter 20. It should be noted, however, that the structure of the hub 3 can be properly modified depending on the outer diameter of the tube body 2, part of the body to which the catheter is to be inserted, and the like.

As explained in the foregoing, the present invention provides a tube for introducing a tubular or a wire-shaped medical device such as a catheter and a wire guide into a living body, said tube for introducing medical devices being such which first introduces the medical device by bearing the device into the hollow portion thereof and which is then removed by tearing off, characterized by that it is made of a silicone - polyimide copolymer resin capable of being easily rendered molecular orientation along the extruded direction thereof. The use of this particular resin readily enables production resin tubes reduced in mechanical strength of the tube wall along the tube periphery by extruding the resin though a commonly employed extrusion molding machine without involving any special apparatus. Thus, tubes which can be readily parted along the longitudinal direction thereof are produced in good economy.

Furthermore, concerning the fact that the conventional tubes had to be torn only from a restricted portion and that they thereby required a step of positioning the hub at its attachment to the tube end, the tube according to the present invention is considerably improved in operability, since the tube parting can be initiated from any part as desired in the tube periphery.

A case of introducing a catheter was explained in the foregoing example, but it should be noted that the tube according to the invention is applicable to other tubular or wire-shaped medical devices, such as wire guides and by-pass tubes, and that the tube can be used for introducing medical devices into tubular organs and celoms other than blood vessels as well.

While the invention has been described in detail and with reference to specific embodiments thereof, it will be apparent to one skilled in the art that various changes and modifications can be made therein without departing from the spirit and scope thereof.

**Claims**

1. A Tube (2) for introducing percutaneously a tubular or a wire-shaped medical device such as a catheter (20) into a living body,

said tube (2) for introducing medical devices being such which first introduces the medical device by bearing the device into the hollow portion thereof and which is then removed by tearing off, and

said tube (2) being characterized by that it is made of a silicone-polyimide copolymer resin.

2. Tube according to claim 1, characterized in that it is made of a material having longitudinally oriented molecular chains.

3. Tube according to claim 1 or 2, characterized in that it is made of extruded material which has been stretched immediately after discharge from the extruder die.

Fig. 1

Fig. 2

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y | US-A-4 473 067 (SCHIFF)<br>* column 1, line 68 - column 2, line 2; figures 3,5 *<br><br>--- | 1,2 | A61M25/06 |
| Y | US-A-4 555 398 (ODA)<br>* column 3, line 13 - line 18 *<br><br>--- | 1,2 | |
| A | US-E-31 855 (OSBORNE)<br>* column 2, line 44 - line 47 *<br>* column 5, line 28 - line 33; figure 1 *<br><br>----- | 2,3 | |
| | | | TECHNICAL FIELDS SEARCHED (Int. Cl.5) |
| | | | A61M |

The present search report has been drawn up for all claims

| Place of search | Date of completion of the search | Examiner |
|---|---|---|
| THE HAGUE | 14 FEBRUARY 1992 | SEDY R. |

EPO FORM 1503 03.82 (P0401)